# EUROPEAN PATENT SPECIFICATION

(11) **EP 0 643 584 B1**
(45) Date of publication and mention of the grant of the patent: **15.03.2000**
(21) Application number: 93914191.7
(22) Date of filing: 27.05.1993
(51) Int. Cl.: A61K 38/49, C07K 14/00

(54) **DELIVERY OF A CYTOTOXIC COMPOUND TO A CANCER CELL USING A PATHWAY OF PLASMINOGEN ACTIVATOR MATERIAL**
BEREITSTELLUNG EINER FÜR KREBSZELLEN CYTOTOXISCHEN VERBINDUNG UNTER VERWENDUNG DES WEGES VON PLASMINOGENAKTIVATORMATERIAL
APPORT D'UN COMPOSE CYTOTOXIQUE DANS UNE CELLULE CANCEREUSE PAR L'INTERMEDIAIRE D'UN MATERIAU ACTIVATEUR DU PLASMINOGENE

(30) Priority: 28.05.1992 US 889783; 10.05.1993 US 59813
(43) Date of publication of application: 22.03.1995
(73) Proprietor: THE UNIVERSITY OF TOLEDO, Toledo, OH 43606 (US)
(72) Inventor: JANKUN, Jerzy, Sylvania, OH 43560 (US); HART, Richard, Greenwich, CT 06836-1165 (US)
(74) Representative: Dempster, Benjamin John Naftel
(86) International application number: US9305034
(87) International publication number: WO9324141

(56) References cited:
- WO-A-90/12091
- US-A- 5 087 616
- CANCER RES. (1992), 52(20), 5829-32 CODEN: CNREA8;ISSN: 0008-5472, 1992 JANKUN, JERZY 'Antitumor activity of the type 1 plasminogen activator inhibitor and cytotoxic conjugate in vitro'
- The EMBO Journal, Volume 7, No. 4, issued 1990, M.V. CUBELLIS et al., "Receptor-mediated Internalization and Degradation of Urokinase is Caused by its Specific Inhibitor PAI-1", pages 1079-1085, Abstract.
- Cancer Research, Volume 50, issued 01 August 1990, M.S. BAKER et al., "Inhibition of Cancer Cell Urokinase Plasminogen Activator by its Specific Inhibitoir PAI-2 and Subsequent Effects on Extracellular Matrix Degradation", pages 4676-4684, Abstract.

## Description

The present invention relates to a use of a cytotoxic material coupled to plasminogen activator inhibitor material for the manufacture of a medicament for the treatment of cancer cells containing a Urokinase plasminogen activator (uPA) bound to the surface by uPA receptor, characterised in that the plasminogen activator inhibitor (PAI) is PMI-1 or PAI-2, or recombinant forms or derivatives thereof, bound to a carrier.

### BACKGROUND OF THE INVENTION

Cytostatic drugs have been in clinical use as anticancer agents. Cytotoxic or cytostatic compounds such as methotrexate, dexorubicin, cis-platinum and others have high cytotoxicity but generally have low target selectivity (low efficiency in hitting the cancer cell target).

In modern oncology there is a consensus that the trend of development of therapeuticals shifts increasingly from the emphasis on the maximal cytotoxicity toward the maximal selectivity. Cytostatic drugs have been in a clinical use as anticancer agents for about half a century now. Over the years, a number of cytotoxic or cytostatic compounds have been developed and clinically applied in tumor therapy (methotrexate, dexorubicin, cis-platinum and others). The listed above agents have in common their high cytotoxicity, though their therapeutic efficacy is limited by their relatively low target selectivity. Only the fact, that the tumor cells grow and metabolize a lot faster than most healthy tissues, makes chemotherapy of tumor work at all.

Around the turn of the century, Paul Ehrlich first propagated the idea of targeted drug delivery. He referred to targeted drug as a magic bullet that would seek out its target and hit there only without damaging any other tissue. If one can make the cytostatic compound more tumor specific by this method, one would automatically increase the therapeutic index and thus the efficacy of the treatment. It is therefore no surprise that the antibody-drug conjugates were and are considered a possible way to increase drastically selectivity of the treatment. There is, so far, no magic bullet-like target seeking ability for the antibody; the only way an antibody reaches its target is by passive transport with blood or lymphatic fluid, However, an antibody does not actively seeks its target, the only reason to use it in localization on the target cells is the high affinity constant of the antibody-antigen reaction. Thus, the dilution effects, accessibility of a tumor location by these fluids, and unspecific F_{c} receptor binding (in the liver) diminish the amount of immunoconjugates reaching the target. Even once an immunoconjugate has reached the tumor it still has to penetrate into the malignant cells. Therefore, while the antibody-mediated diagnosis and the therapy of tumors are probably the closest medical equivalent to the magic bullet concept, there was no way yet to achieve active transport of the immunoconjugates into a target cell (except, perhaps, for some special cases like cells of the RES system (reticulo-endothelial system), that actively take up the macromolecular compounds as a part of their natural function). In addition, most monoclonal antibodies applied in the immunoconjugate research and clinical application are of murine origin. As a consequence, every patient treated with the murine immunoconjugates will develop human antimurine antibodies. This leads to negative side effects from allergic reactions to anaphylactic shock. The development of the human-human antibodies, humanized antibodies, and immunoreactive peptides will probably at some point in the future solve the anti-mouse response problems.

The immunoconjugate concept is based on the idea that the localization ability of an antibody is utilized to deliver a therapeutic (or diagnostic) compound to the tumor. Thus, this compound has to be attached to the antibody in a way that can be delivered to the tumor without being removed from the antibody before reaching the target. As every protein, antibodies contain the amino acids that have functional groups in their side chains, carbohydrate residues (so called F part of the antibody), and charged amino acids. All of them are potential coupling sites. The coupling protocols of the antibodies and the therapeutic agents vary greatly depending on the nature of the coupling sites of the antibody and drug. However, most cases involve a chemical modification of both agents, which in most sever cases, can lead to the complete inactivation of the antibodies.

The conjugation of the known cytotoxic compounds to the antibodies under no circumstances limit potential use of other compounds. As a matter of fact, more papers were published on research done on the toxin-conjugated antibodies than known drug immunoconjugates. Virtually all available toxin can be attached to antibodies, but the most frequently used are: ricin (from plants), diphtheria toxin (from microorganism), or cobra venom factor (from animals). One of the prime objectives in the development of any chemotherapeutic agents is the maximum toxic effect with minimal dosage. All mentioned toxins are very toxic in very low concentrations.

In the toxin research, it has been found that most toxin discussed here, (ribosome inactivating proteins like ricin, abrin, and with different mechanism of toxicity like diphtheria toxin and cholera toxin) consist of two protein chains, that play different roles in the cytotoxic process. The so-called A-chain causes cytotoxic action. The B-chain binds specifically to galactose units on glycoproteins and glycolipids on cell membranes (present on basically all cells of animals and humans) and then acts to channel A-chain into the cells. Thus with modifications to the toxin to remove the B-chain or to moderate its galactose-binding action, a virtually non-toxic toxin could be prepared and conjugated to the antibody. However, the attachment of the antibody to the cell surface alone does not yet guarantee cell uptake of the conjugate or at least the cytotoxic moiety. Introduction of B-chain of toxin will take action to facilitate entry of A-chain into cytosol but at the same time will increase unspecific binding of the entire complex.

A new concept in the treatment of cancer is a development of immunotargeted particles. The use of these particles in tumor therapy is based on the thought that particles (either solid or hollow) can hold a relatively large amount of a cytotoxic compound, thus creating a high drug concentration at the tumor site, if delivered selectively. So far the most extensively studied systems of this kind are liposomes and polymeric micro/nanoparticles.

Liposomes are small vesicles that consist of one or more layers of amphophilic molecules (e.g., phospholipids). These compounds have a hydrophilic (charged) group at one end and a hydrophobic tail at the other. consequently, when formed from an emulsion of an organic and an aqueous phase, these vesicles form by alignment of two layers of hydrophobic groups next to each other, thus yielding a membrane with hydrophobic character at its inner and outer surface. An aqueous solution enclosed in such vesicles can contain hydrophilic drugs, while the bilayer itself can contain hydrophobic drugs. Antibodies can be incorporated into the membrane of liposomes, yielding immunoliposomes. The most important drawback of liposomal carriers is the fact, that not only immunoliposomes will be attached to a tumor cell surface remain outside the cell, but also, this liposome will have to be brought to release its load of cytotoxic compound at that point, but not in circulation.

Other, particles used a drug carriers, mostly polymeric, rarely have been described in the connection with antibodies as a targeting device. Gold nanoparticles have some potential to be used as carriers in cancer treatment. Gold spheres are produced by chemical reduction of tetrachloroauric acid. They are formed by condensation of metallic gold from a super saturated solution created by reduction of Au⁺³ present in the tetrachloroauric acid. If the reduction is rapid, many small gold particles are formed. By regulating the conditions of the reduction one can adjust the size of the gold spheres between 2-150 nm. Gold particles of this size can absorb the proteins. It is assumed that protein absorption is due to the electrostatic interactions between the negatively charged surface of gold particles and positively charged groups of proteins. This non-covalent binding of proteins to gold sphere interferes only minimally with the reactivity of the proteins. However, since the forces involved in complex formation are non-covalent, the most critical question concerns the stability of such complexes. It is generally assumed that proteins are irreversibly absorbed onto colloidal gold. There is some evidence to support this assumption. The iodinate I¹²⁵ tetanus toxin-gold complex incubated with rat serum lost only 9% radioactivity after 14h. Most authors agree that storage of the protein-gold complexes at 4°C is adequate. It has been found that colloidal gold dialyzed to 45% glycerol in PBS may be stored at -18°C.

There is a need to provide a method of delivering cytotoxic compounds to the cancer cell target in an amount effective to retard or halt the cancer cell growth without also destroying normal cells. In addition, there is a need to provide a way of not only locating the cancer cell target, but also to get the cytotoxic compound inside the cancer cell.

### OBJECTS OF THE INVENTION

It is an object of the present invention to provide a use of a cytotoxic material coupled to plasminogen activator inhibitor material for the manufacture of a medicament for the treatment of cancer cells containing a Urokinase plasminogen activator (uPA) bound to the surface by uPA receptor, characterised in that the plasminogen activator inhibitor (PAI) is PAI-1 or PAI-2, or recombinant forms or derivatives thereof, bound to a carrier.

These and other objects will be apparent from the specification that follows and the appended claims.

### SUMMARY OF THE INVENTION

The present invention provides a use of a cytotoxic material coupled to plasminogen activator inhibitor material for the manufacture of a medicament for the treatment of cancer cells containing a Urokinase plasminogen activator (uPA) bound to the surface by uPA receptor, characterised in that the plasminogen activator inhibitor (PAI) is PAI-1 or PAI-2, or recombinant forms or derivatives thereof, bound to a carrier.

Preferred cytotoxic compounds are: methotextrate, cobra venom, A-chain cholera toxin, A-chain ricin, and saporin.

Suitable carriers are gold particles, polymeric nanoparticles, and liposomes. The average diameter of the particle is generally about 1 to 150 nm and preferably about 10 to 15 nm.

The reaction product of the coupling step is made generally at a temperature of about 4° to 40°C and preferably about 8 to 20°C for a time generally of about 1 second to 60 minutes and preferably about 2 to 30 seconds.

Using the plasminogen material pathway, the PAI-1 toxic compound reaction product is not only delivered to the cancer cell but the reaction product couples with the uPA/uPAR on the cell surface whereupon the resultant reaction product/uPA/uPAR complex is efficiently internalized within the cell where the complex is degraded to deliver the cytotoxic compound within the cell to stop, slow down or retard the cancer growth.

In a number of different tumor systems, direct correlation has been found between levels of urokinase plasminogen activator (uPA) activity and metastatic potential. Since the discovery of the uPA cell surface receptor, this correlation between receptor-bound or cell associated uPA and malignancy has been well documented. Thus, in human breast cancers, high levels of uPA are associated with both shortened disease free interval and shortened overall survival. In human colonic cancer, total uPA activity has been shown to correlate with local invasion and metastasis. Finally, in human lung cancers, levels of mRNA for uPA correlate with invasion to local lymph nodes. A number of studies have shown that administration of serine protease inhibitors to animal prevented or decreased metastasis formation. Also, the administration of uPA was found to enhance the formation of pulmonary metastasis, but in presence of uPA inhibitors the enhancing effect was abolished.

The uPA is produced by cells in a form of inactive, single chain proenzyme (pro-uPA). Conversion of pro-uPA to active two-chain uPA is performed by catalytic amounts of plasmin. This conversion provides active uPA, catalyzing transformation of plasminogen to plasmin and enables an autocatalytic acceleration of uPA formation. The uPA can bee secreted to extracellular space or can be bound by the uPA receptor (uPAR) on the cell surface. Binding is rapid, saturable, and with high affinity of 10⁻¹⁰M. The bound enzyme is not internalized or rapidly degraded. Binding of uPA to the receptor does not involve the catalatic site of uPA but it occurs at EGF (epidermal growth factor) Like domain. Receptor-bound uPA converts plasminogen to plasmin, and creates an area of localized and high proteolytic activity on the cancer cell surface. The receptor-bound uPA can bind plasminogen activator inhibitor (PAI-1). The binding is strong (10⁻¹³M) and only then, after binding PAI-1 triggers internalization of the PAI-1/uPA/uPAR complex. The complex is degraded and the receptor is probably recycled, and products of degradation are released to the cytosol.

It has been determined that uPA is commonly expressed on the cell surface of the cancer cells in higher amounts than on the normal ones. The uPA serves as the tumor associated antigen to distinguish between malignant and normal cells on the molecular level. To locate the cancer cells the PAI-1, a high affinity binding protein, will be used. The mechanism action is as follows:
a. PAI-1 coupled to carrier and toxin binds in high numbers to the cancer cells.
b. uPAR/uPA/PAI-1/carrier/toxin complex will be internalized by endocytosis.
c. in the lysosome uPAR/uPA/PAI-1/carrier/toxin complex will be degraded and its contents, including toxin, will be released to the cytosol.

Recently, high potency bacterial or plant toxins have increasingly been used as the toxic moieties for attachment to monoclonal antibodies in construction of immunotoxins. To protect the cells from unspecific binding the A-chain containing the enzymatic (cytotoxic), but not binding activity have been used. The most commonly used toxins are: ricin and diphtheria toxin. They are very toxic substances, and because of concern for safety they were excluded from an early preliminary study. Instead, the A-chain of cholera toxin was used. Under ordinary conditions cholera toxin containing A- and B-chains binds to cell surface to the GM1 receptor. Then the A-chain enters the cell. Once inside the cell, the A subunit functions enzymatically to initiate the chain of events that poisons the cell by permanently turning ON the regulatory proteins known as G proteins. That leads to overproduction of cyclic AMP (adenosine monophosphate), which stimulates the enzyme cyclic AMP-dependent kinase, ultimately causing the cells to secrete copious amounts of water. Under in vitro condition this leads to death of the cells. In the early study, the role of the B-chain is to safely substitute for the PAI-1. The PAI-1 recognizes, binds, and internalizes toxin by the plasminogen activator pathway. Colloidal gold was used as a carrier for PAI-1 and cholera toxin. The binding to the nanospheres of gold (5-15nm) is permanent, and appears to preserve biological properties of bond proteins. Colloidal gold, was used for the preliminary study. The gold can be replaced, if needed, by other carriers such as liposomes, polymeric nanoparticles, or others.

The following illustrates the colloidal gold preparation in the preliminary study. In some experiments colloidal gold (8-12nm), from Sigma was used (A-5179). In others the gold was prepared by method described by Beesley. In brief, the 80 ml of 0.01% tetrachloroauric acid was reduced by 4 ml of 1% trisodium citrate with 0.03 ml of 1% tannic acid at 60°C. Next the solution was boiled for 2 minutes and colloidal gold was formed. Yielding under these conditions gold particles with diameter 12 nm.

The binding of proteins to colloidal gold nanoparticles was accomplished when 300 µL of colloidal gold was mixed with 50 µL of PAI-1 solution (#1090 from American Diagnostica, Inc.). After 30 minutes, particles were tested for the presence of biologically active PAI-1 protein. In brief, the murine monoclonal antibody (# 380 from American Diagnostica, Inc.) was absorbed by the surface of microtiter plates, 96 wells. After washing of unbound excess of anti PAI-1, colloidal gold with bounded PAI-1 was incubated in a series of dilutions at 37°C for 30 minutes (dilution: 1:1, 1:2, 1:4, 1:8, 1:16, 1:32, 1:64). As a negative control, colloidal gold without PAI-1 has been used. Next, the wells were washed 3 times with PBS and silver enhancer was added. After 5 minutes the silver solution was removed, the wells were washed and 1% of sodium thiosulfate was added to fix the metallic silver. Binding of the A-chain of cholera toxin was done basically in the same way as binding of PAI-1. The 350 µL of the colloidal gold solution bearing PAI-1 was mixed with 50 µL of the A-chain cholera toxin.

The cell and cell culture conditions used to determine the binding of the gold particles are as follows:

The HT1080 fibrosarcoma cells, expressing receptor bound uPA (13.4 PU/10⁶ cells) and KD normal fibroblast expressing very low amount receptor bound uPA (less than 0.1 PU/10⁶ cell), were routinely grown in αMEM medium, supplemented with 10% new born calf serum, L-streptomycin and penicillin at 37°C in humidified incubator (5% CO₂, 95% air). Both cell lines were obtained from American Type Culture Collections. The cytotoxicity study was done in serum free conditions. The cells were incubated for 48 hours in various concentrations of colloidal gold solutions. In the end of incubation period cells were washed with PBS (phosphate buffered saline - Ca⁺² and Mg⁺² free), trypsynized and counted.

A scanning electron microscope study was conducted on the cells incubated with PAI-1/A-chain cholera toxin/colloidal gold. The cells, after removing the medium, were washed 3 times with cold PBS, fixed with 50% ethanol, and dehydrated by wash of 75%, 85%, 95%, and 100% ethanol. Next they were dried and mounted to be shadowed with carbon or gold.

A fluorescence-microscope study was made using labeled PAI-1. The PAI-1 was dialyzed against PBS and 0.01 mg/ml of TRITC (tetramethyl rhodamine isothiocyanate) at 4°C overnight. Next, TRITC labeled PAI-1 was dialyzed for 3 days with several changes of PBS at 4°C. The TRITC labeled protein was then non-covalently bound to the colloidal gold. Cells were grown on the glass cover slips and next incubated with TRITC/PAI-1/A-chain cholera toxin/gold complexes for 5-60 minutes. Cells were washed 5 times with PBS fixed in 50% ethanol and dehydrated as described before. Air dried cells were stored until needed. Before microscope examination cells were rehydrated in PBS. Cover slips were mounted on microscope slides using mounting medium (FITC-Guard from Testog Inc.) and sealed with nail polish.

The minimal amount of protein needed to stabilize the gold sol was determined. The stability of the gold sol in water is maintained by repulsion of the electrostatic charges on each gold particle. The addition of the strong electrolytes compresses the ion layers around each particle and allows the particles to approach closely to each other. If a critical distance is reached the particles aggregate and flocculation of the colloidal gold occurs. Proteins absorbed on the surface of the gold inhibit the electrolyte-induced coagulation of the gold. This characteristic has been exploited in the form of a simple titration to determine the minimum amount of BSA needed to stabilize the gold particles. Flocculation can be judged by the change of the color from red (stabilized gold) to blue (flocculated gold).

The presence of PAI-1 on the surface of colloidal gold was determined when the colloidal gold particles bearing the PAI-1 were incubated in microtiter plates coated with MoA against PAI-1, a very light pink color of colloidal gold on first two wells has been observed. After silver enhancing, the gray-metallic color of metallic silver has been detected on all, except the control wells, which were negative. As expected, the intensity of the color produced by silver enhancement was proportional to the amount of antigen present in each well.

The presence of the PAI-1/gold/toxin complex on the surface of the cells was determined as follows:

The gold particles complexed with proteins (PAI-1 and A-chain of cholera toxin) are relatively large. This big size (in scale of the living cell alone) does not guarantee that particle can easily carry 10,000 particles of each absorbed proteins. Due to three dimensional structure, only a few will be involved in the process of binding to the cell surface. The binding is sufficiently strong enough to keep the gold particle in the place, and is determined by a scanning electron microscope study. Careful examination showed numerous bright spots (bright spots are gold particles or gold aggregates) on the surface of the HT1080 cells. This cancer cell line is expressing high activity of the uPA on the cell surface showing high binding. The size of observed spots corresponds to expected size of gold particles or cluster of gold particles presumably undergoing the process of endocytosis. Quite contrary, the KD-normal fibroblastic cells expressing low activity of uPA on the cell surface, as we expected, showed much lower number of gold particles. This demonstrates that PAI-1-uPA bound is strong enough to keep PAI-1 and toxin bearing gold particle on the surface of the living cell and that gold particle bearing the PAI-1 and toxin can undergo the process of internalization.

Internalized TRITC labeled proteins were detected by fluorescence microscopy. The living HT1080 cells were incubated with gold sol bearing TRITC labeled PAI-1 and A-chain of cholera toxin. The cells were exposed to gold sol for 5, 10, 30 and 60 minutes in serum containing and serum-free medium. The cells in serum free medium showed slightly higher fluorescence. No significant differences were visible between 5 and 60 minutes incubation indicating that process of internalization is relatively quick. The fluorescence has been observed in cytoplasm, and higher intensity has been observed in the perinuclear region, where lysosomes have been shown to localize. This demonstrates that process of internalization is executed by endocytosis.

The cytotoxic activity of PAI-1/A-chain/gold complex was determined. Experiments described before explicitly demonstrated that besides the size and non-covalent binding to the colloidal gold the PAI-1 is able to deliver the gold sol into the cells. The A-chain cholera toxin was shown to survive the somewhat harsh condition of lysosomes (pH 5.0 and proteolytic enzymes), leave lysosomes and still effectively poison the cells. Previously described toxins are entering the cells in this way, but A-chain cholera toxin is lacking the B-chain. This modification can have an effect on the ability of the A-chain to persist in undernaturated form through lysosome pathway. It may not. To rigorously determine this, the HT1080 and KD cells are poisoned for 48 hours in serum free conditions. Serum contains many, very often undefined, proteins. Some of them can bind in gold particles.

The localization and killing or reducing the growth of cancer cells was determined. Cells were divided into three groups. The first contained cells treated with PAI-1/A-chain cholera toxin/gold complex with following dilutions - 1:10, 1:20, 1:40, 1:80, 1:160, 1:320, 1:640, 1:1280. It is expected that differences will be demonstrated in the cell killing between cancer and normal ones. The second group contained cells treated with A-chain cholera toxin/gold complex. It is expected that this complex would not be internalized into the cells, since it was not equipped with molecules responsible for it. No cell killing would be observed. The third group contained cells treated with PAI-1/A-chain cholera toxin/gold complex and treated with excess unbounded PAI-1 (estimated amount - 50 times more than cytotoxic component). We expected that high amount of free PAI-1 will saturate uPAR/uPA complex on the cell surface and abolish the cytotoxic effect of the PAI-1/A-chain cholera toxin/gold complex. The results show the therapeutic value of finding and killing cancer cells by the PAI-1 pathway the first group showing the localizing or finding action, followed by internalization and killing the cancer cells or reducing their growth.

The plasminogen pathway material can be a plasminogen activator inhibitor PAI-2. PAI-2 can be substituted in whole or part for the PAI-1 pathway material.

Another pathway material or cancer cell localizing material is penicillin that provides in vitro inhibition of urokinase inhibitor (uPA) and can be used to couple to the cytotoxic compound.

The PAI-2 is a known material as is penicillin. Background publications that describe the inhibition of urokinase by penicillin and the PAI-2 pathway material are as follows:
1. "In Vitro Inhibition of Urokinase by Penicillin", Abd Al-Roof Higazi and Michael Mayer, 1988.
2. "The Receptor for Urokinase Type Plasminogen Activator Polarizes Expression of the Protease to the Leading Edge of Migrating Monocytes and Promotes Degradation of Enzyme Inhibitor Complexes", Estreicher, et al., August, 1990
3. "Biochemical and Biological Aspects of the Plasminogen Activation System", Michael Mayer, June, 1990.

## Claims

1. The use of a cytotoxic material coupled to plasminogen activator inhibitor material for the manufacture of a medicament for the treatment of cancer cells containing a Urokinase plasminogen activator (uPA) bound to the surface by uPA receptor, characterised in that the plasminogen activator inhibitor (PAI) is PAI-1 or PAI-2, or recombinant forms or derivatives thereof, bound to a carrier.

2. The use of a cytotoxic material according to claim 1, characterised in that the carrier is a gold particle, nanoparticle or a liposome.

3. The use of a cytotoxic material according to claim 1 or claim 2 characterised in that the cytotoxic compound is methotrexate, a derivative of methotrexate, cobra venom, A-chain cholera toxin, A-chain ricin or saporin.

## Patentansprüche

1. Verwendung eines zytotoxischen Materials, das mit einem PlasminogenaktivatorHemmunungsmaterial gekoppelt ist, zur Herstellung eines Medikaments für die Behandlung von Krebszellen, das eine Urokinase-Plasminogenaktivator(uPA)-Bindung an die Oberfläche durch einen uPA-Rezeptor enthält, dadurch gekennzeichnet, daß der Plasminogenaktivatorhemmer (PAI) aus PAI-1 oder PAI-2 oder rekombinanten Formen oder Derivaten derselben besteht, die an einen Träger gebunden sind.

2. Verwendung eines zytotoxischen Materials nach Anspruch 1, dadurch gekennzeichnet, daß der Träger ein Goldpartikel, ein Nanopartikel oder ein Liposom ist.

3. Verwendung eines zytotoxischen Materials nach Anspruch 1 oder Anspruch 2, dadurch gekennzeichnet, daß die zytotoxische Verbindung Methotrexat, ein Derivat von Methotrexat, Cobravenom, ein A-Ketten-Choleragen, ein A-Ketten-Rizin oder ein Saporin ist.

## Revendications

1. Utilisation d'un matériau cytotoxique couplé à un matériau inhibiteur activateur plasminogène pour la fabrication d'un médicament destiné au traitement de cellules cancéreuses contenant un activateur plasminogène Urokinase (uPA) lié à la surface par un récepteur uPA, caractérisé en ce que l'inhibiteur activateur plasminogène (PAI) est un PAI-1 ou bien un PAI-2, ou encore des formes recombinées ou des dérivés de ces derniers, liés à un porteur.

2. Utilisation d'un matériau cytotoxique selon la revendication 1, caractérisé en ce que le porteur est un liposome, une particule ou bien une nanoparticule d'or.

3. Utilisation d'un matériau cytotoxique selon la revendication 1 ou la revendication 2, caractérisé en ce que le composé cytotoxique est du méthotrexate, un dérivé de méthotrexate, du venin de cobra, une toxine de choléra de chaîne A, une saporine ou ricine de chaîne A.
